(19) Europäisches Patentamt
European Patent Office
Office européen des brevets

(11) Veröffentlichungsnummer: **0 048 371**
**B1**

(12) # EUROPÄISCHE PATENTSCHRIFT

(45) Veröffentlichungstag der Patentschrift: **28.12.83**

(51) Int. Cl.³: **C 07 C125/06**, C 07 C118/00

(21) Anmeldenummer: **81107012.7**

(22) Anmeldetag: **07.09.81**

(54) **Verfahren zur Herstellung von N,O-disubstituierten Urethanen.**

(30) Priorität: **19.09.80 DE 3035354**

(43) Veröffentlichungstag der Anmeldung: **31.03.82 Patentblatt 82/13**

(45) Bekanntmachung des Hinweises auf die Patenterteilung: **28.12.83 Patentblatt 83/52**

(84) Benannte Vertragsstaaten: **BE CH DE FR GB IT LI NL**

(56) Entgegenhaltungen:
**DE-A- 1 944 719**
**DE-A- 2 004 606**
**DE-A- 2 530 001**
**DE-B- 2 716 540**
**US-A- 3 763 217**

(73) Patentinhaber: **BAYER AG**
**Zentralbereich Patente, Marken und Lizenzen**
**D-5090 Leverkusen 1, Bayerwerk (DE)**

(72) Erfinder: **Buysch, Hans-Josef, Dr.**
**Brandenburger Strasse 28**
**D-4150 Krefeld (DE)**
Erfinder: **Krimm, Heinrich, Dr,**
**Heyenbaumstrasse 65**
**D-4150 Krefeld 1 (DE)**
Erfinder: **Richter, Wolfgang, Dr.**
**Scheibler Strasse 111**
**D-4150 Krefeld (DE)**

**EP 0 048 371 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur Herstellung von N,O-disubstituierten Urethanen

Die vorliegende Erfindung betrifft ein verbessertes Verfahren zur Herstellung von N,O-disubstituierten Urethanen durch Umsetzung von primären Aminen mit Dialkylcarbonaten.

Es ist bekannt, Amine mit Kohlensäureestern zu Urethanen umzusetzen. Nach USP 3 763 217 verwendet man dazu als Katalysatoren Lewissäuren und Uranverbindungen. Diese Verbindungen katalysieren jedoch neben der Urethanbildung die Alkylierung der Amine durch die Kohlensäureester, so daß erhebliche Mengen an N-alkylierten Aminen entstehen. Diese Nebenreaktion nimmt mit der Temperatur rasch zu. Eine andere Nebenreaktion, die Harnstoffbildung, nimmt mit der Reaktionszeit zu. Daher steht für dieses Verfahren nur ein sehr begrenzter Zeit- und Temperaturspielraum zur Verfügung.

Die eingesetzten Katalysatormengen betragen in der Regel um 10 Gew.-%, oft auch mehr. Da sie als Lewissäuren mit den Aminen Komplexe bilden, ist es praktisch unmöglich, die Katalysatoren nach der Aufarbeitung unversehrt zurückzugewinnen.

Einigermaßen zufriedenstellende Ausbeuten scheinen nur Urantrioxid ($UO_3$) und Urantetrachlorid ($UCl_4$) unter Anwendung von ca. 10 Gew.-% bei 80 °C und langen Reaktionszeiten zu liefern. Uranverbindungen sind jedoch wegen der Gefahr radioaktiver Verseuchung und der daher erforderlichen umfangreichen und kostspieligen Sicherheitsvorkehrungen für eine technische Verwendung im vorliegenden Prozeß indiskutabel.

Die DE-OS 2 004 606 lehrt die Umsetzung von N,N'-disubstituierten Harnstoffen mit Dialkylcarbonaten in Gegenwart von Basen als Katalysator zu N,O-disubstituierten Urethanen. Der naheliegende Gedanke, auch beim Verfahren der US-PS 3 763 217 die in der DE-OS 2 004 606 erwähnten Katalysatoren einzusetzen, um so die Bildung von unerwünschten Harnstoffen zu vermeiden, führt jedoch nicht zum angestreben Ergebnis, da sich die in der DE-OS 2 004 606 empfohlenen Basen nicht als Katalysatoren für das Verfahren gemäß US-PS 3 763 217 eignen. Die Basen sind vielmehr katalytisch völlig wirkungslos. Die DE-AS 2 716 540 befaßt sich mit der Herstellung von Urethanen aus N-Acylarylaminen und Dialkylcarbonaten, wobei als Katalysatoren Phenolate bzw. Alkoholate des Titans bzw. Aluminiums zum Einsatz gelangen. Die Tatsache, daß die in der DE-AS 2 716 540 beschriebene Umsetzung mit der nachstehend näher beschriebenen erfindungsgemäßen Umsetzung in keinen näheren Zusammenhang gebracht werden kann, ergibt sich schon allein aus dem Umstand, daß die in der Vorveröffentlichung erwähnten Katalysatoren z. T. (z. B. Titanalkoholate) auch beim erfindungsgemäßen Verfahren als Katalysator brauchbar sind, zum Teil jedoch (z. B. Aluminiumalkoholate) völlig unbrauchbar sind, d. h. die bereits aus der US-PS 3 763 217 bekannte Eigenschaft aufweisen, unerwünschte Nebenreaktion (Harnstoffbildung) zu katalysieren. Auch die DE-AS 2 716 540 kann daher keinen Hinweis vermitteln, wie die genannten Schwierigkeiten in Zusammenhang mit US-PS 3 763 217 überwunden werden könnten.

Ein anderes Verfahren zur Herstellung von N-Aryl-O-alkylurethanen wird unter Verwendung von Alkalimetallbesonders Na-Verbindungen bei der Umsetzungen aromatischer Amine mit Dialkylcarbonaten durchgeführt (offengelegte jap. Patentanmeldung 090 478). Die Alkalimetallverbindungen werden allerdings in mindestens äquimolarer Menge bezogen auf das Amin eingesetzt, bei der Aufarbeitung durch Neutralisation mit Säuren entfernt und sind damit verloren. Deshalb läßt sich auch dieser Prozeß nicht großtechnisch gestalten.

Wie nun überraschenderweise gefunden wurde, eignen sich die nachstehend näher beschriebenen Verbindungen des Bleis, Titans, Zinks und Zirkons hervorragend als Katalysatoren für die Herstellung von N,O-disubstituierten Urethanen durch Umsetzung der entsprechenden primären Amine mit den entsprechenden Dialkylcarbonaten. Bei Verwendung dieser erfindungsgemäßen Katalysatoren entstehen im Gegensatz zum Verfahren der US-PS 3 763 217 keine N-Alkylamine. Ohne Gefahr von Nebenreaktionen können höhere Temperaturen angewandt und damit auch kürzere Reaktionszeiten erzielt werden. Die Reaktion kann bis zum vollständigen Umsatz des Amins getrieben werden, ohne dabei die Harnstoffbildung zu fördern.

Gegenstand der vorliegenden Erfindung ist somit ein Verfahren zur Herstellung von N,O-disubstituierten Urethanen durch Umsetzung von primären Aminen mit Dialkylcarbonaten in Gegenwart von Katalysatoren, dadurch gekennzeichnet, daß man als Katalysatoren neutrale oder basische anorganische oder organische Verbindungen des Bleis, Titans, Zinks oder Zirkons verwendet.

Für das erfindungsgemäße Verfahren geeignete Amine sind beliebige organische Verbindungen, die mindestens eine primäre Aminogruppe aufweisen und ansonsten unter den Reaktionsbedingungen inert sind. Vorzugsweise werden beim erfindungsgemäßen Verfahren derartige organische Verbindungen eingesetzt, die ausschließlich aromatisch gebundene Aminogruppen aufweisen. Besonders bevorzugte Ausgangsmaterialien des erfindungsgemäßen Verfahrens sind Verbindungen der Formel

$$R^1\text{—}(NH_2)_n$$

bzw. Gemische derartiger Verbindungen, wobei

$R^1$ für einen gegebenenfalls Alkyl- und/oder Halogen-Substituenten und/oder Alkylen-Brücken, insbesondere Methylen-Brücken aufweisenden aromatischen Kohlenwasserstoffrest mit insgesamt 6-15 Kohlenstoffatomen steht und

n 1 oder 2 bedeutet.

Beispiele geeigneter Amine sind Anilin, o-, m-, p-Toluidin, o-, m-, p-Chloranilin, o-, m-, p-Bromanilin, o-, m-, p-Trifluormethylanilin, 2,4-, 2,6-, 3,4- und 3,5-Dimethyl-, -Dichlor-, -Dibrom- und -Diethylanilin, p-tert. Butylanilin, m- und p-Phenylendiamin, 2,4- und 2,6-Diaminotoluol, α- und β-Naphthylamin, 1,4-, 1,5-, 2,6- und 2,7-Diaminonaphthalin, 2,4'-, 2,2'-, 4,4'-Diaminodiphenylmethan, 3,3'-Dimethyl-4,4'-diaminodiphenylmethan, 3,3'-Dichlor-4,4'-diaminodiphenylmethan, 4,4'-Diaminodiphenyl-propan-2,2 und 4,4'-Diaminodiphenylether, ferner Methylamin, Ethylamin, Isopropylamin, n-Butylamin, Isobutylamin, Cyclohexylamin, Dodecylamin, Tetramethylendiamin-1,4, Hexamethylendiamin-1,6, 2,2,4-Trimethylhexamethylendiamin, Isophorondiamin und 4,4'-Diaminodicyclohexylmethan.

Weitere Ausgangsmaterialien für das erfindungsgemäße Verfahren sind beliebige Dialkylcarbonate, d. h. z. B. Verbindungen der Formel

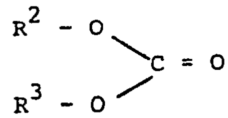

in welcher

$R^2$ und $R^3$ für gleiche oder verschiedene Alkylreste stehen, die vorzugsweise 1-4 Kohlenstoffatome aufweisen.

Besonders bevorzugte Dialkylcarbonate für das erfindungsgemäße Verfahren sind solche Verbindungen der genannten allgemeinen Formel, in welchen $R^2$ und $R^3$ für gleiche gesättigte, unsubstituierte Alkylreste mit 1-4 Kohlenstoffatomen stehen. Grundsätzlich ist es auch möglich, jedoch weniger bevorzugt, bei dem erfindungsgemäßen Verfahren cyclische Carbonate der genannten allgemeinen Formel einzusetzen, für welche $R^2$ und $R^3$ zusammen einen Alkylenrest mit insgesamt 3-6 Kohlenstoffatomen darstellen, der zusammen mit dem Carbonatrest einen heterocyclischen, mindestens 6-gliedrigen Ring bildet. Der Begriff « Dialkylcarbonat » soll im Rahmen der vorliegenden Erfindung somit auch solche cyclischen Carbonate umfassen.

Typische Beispiele von für das erfindungsgemäße Verfahren geeigneten Dialkylcarbonaten sind Dimethyl-, Diethyl-, Di-n-propyl-, Diisopropyl-, Di-n-butyl-, Diisobutyl- und Methyl-ethyl-carbonat sowie ferner cyclische Carbonate wie Trimethylencarbonat oder 2,2-Dimethyltrimethylencarbonat.

Für das erfindungsgemäße Verfahren geeignete Katalysatoren sind beliebige neutrale oder basische Verbindungen des Bleis, Titans, Zinks oder Zirkons. Es handelt sich hierbei um Verbindungen, die in wäßriger Lösung bzw. Suspension neutral oder basisch reagieren. Geeignete Katalysatoren sind vorzugsweise solche, die kein ionogen oder an das Metallatom gebundenes Halogen aufweisen. Es handelt sich somit beispielsweise um die Hydroxide, Oxide, Carbonate, bevorzugt die Salze mit organischen Säuren wie Salze der Sulfonsäuren, z. B. Benzolsulfonsäure, Toluolsulfonsäure, Chlorbenzolsulfonsäure, Phenolsulfonsäure ; Salze der Phosphonsäuren z. B. Benzolphosphonsäure, Toluolphosphonsäure, Chlorbenzolphosphonsäure, Methoxybenzolphosphonsäure, besonders bevorzugt Salze der Carbonsäuren z. B. Formiate, Acetate, Propionate, Butyrate, Laurate, Stearate, Benzoate, Adipate, Maleate, Fumarate, Succinate und Sebacate und Alkoholate wie Methylate, Ethylate, Isopropylate, Butylate und Isoctylate der genannten Metalle.

Die Katalysatoren werden in Mengen von 0,01-6 Gew.-%, bevorzugt 0,05-5 Gew.-%, besonders bevorzugt 0,1-3 Gew.-%, bezogen auf das Reaktionsgemisch, eingesetzt.

Die Reaktionstemperaturen liegen im Bereich von 80-250 °C, bevorzugt 100-200 °C. Das Verfahren kann bei Normaldruck oder erhöhtem Druck durchgeführt werden. Erhöhter Druck ist erforderlich, wenn niedrigsiedende Reaktionspartner bei über ihrem Siedepunkt liegenden Temperaturen umgesetzt werden sollen.

Bei der Durchführung des erfindungsgemäßen Verfahrens kommen die Reaktionspartner im allgemeinen in solchen Mengen zum Einsatz, daß auf jedes Grammäquivalent an Aminogruppen des Amins mindestens 1 Mol an Dialkylcarbonat entfällt. Man kann die Reaktionspartner im stöchiometrischen Verhältnis einsetzen, da infolge glatter Reaktion gemäß der Reaktionsgleichung

$$RNH_2 + (R'O)_2CO \longrightarrow RNHCOOR' + R'{-}OH$$

(die Reste R und R' stehen in dieser lediglich das Reaktionsprinzip erläuternden Gleichung für die indifferenten Reste der Reaktionspartner), das Urethan praktisch das einzige Verfahrensprodukt darstellt. Es kann jedoch auch von Vorteil sein, einen bis zu 30-fachen molaren Überschuß an Kohlensäureestern einzusetzen, da sie als Lösungsmittel für schwerlösliche Ausgangsprodukte bzw. intermediäre gebildete Harnstoffe dienen und zu einem rascheren Ablauf der Reaktion, besonders in der Endphase beitragen. Der sich während der Umsetzung bildende Alkohol wird im allgemeinen fortlaufend während der erfindungsgemäßen Reaktion abdestilliert. Falls Dialkylcarbonate höher siedender Alkohole, insbesondere cyclische Carbonate eingesetzt werden, erfolgt die Abtrennung der Alkoholkomponente vorzugsweise im Anschluß an die erfindungsgemäße Umsetzung durch fraktionierte Destillation. Die erfindungsgemäßen Verfahrensprodukte können im Anschluß an ihre Herstellung destillativ in reiner Form erhalten werden.

Die erfindungsgemäßen Verfahrensprodukte der Formel

$$R^1—(NH—CO—O—R^2)_n$$

in welcher

$R^1$, $R^2$ und n die obengenannte Bedeutung haben, stellen wertvolle Ausgangsmaterialien zur Herstellung der ihnen zugrundeliegenden Isocyanate dar. Hierzu werden die erfindungsgemäßen Verfahrensprodukte in an sich bekannter Weise thermisch in das ihnen zugrundeliegende Isocyanat und den ihnen zugrundeliegenden Alkohol gespalten, worauf sich eine unmittelbare Auftrennung der Spaltprodukte anschließt. Die erfindungsgemäßen Verfahrensprodukte sind außerdem wertvolle Zwischenprodukte zur Herstellung von Schädlingsbekämpfungsmitteln.

Die in den nachstehenden Beispielen gemachten Prozentangaben beziehen sich auf Gewichtsprozente.

Beispiel 1

Ein Gemisch von 31 g (0,33 Mol) Anilin, 118 g (1 Mol) Diethylcarbonat und 1,5 g Titantetrabutylat wird an einer 50 cm langen verspiegelten Vigreuxkolonne zum Sieden erhitzt und Ethanol abdestilliert. Dabei beträgt die Sumpftemperatur 130-140 °C. Nach 6-7 h ist die Umwandlung vollständig und die stöchiometrische Menge Ethanol abgespalten. Durch fraktionierte Destillation im Vakuum wird das Reaktionsgemisch aufgearbeitet. Nach einem Vorlauf von Diethylcarbonat erhält man bei 90-93/0,1 mb 52 g N-Phenyl-O-ethylurethan (FP. 46-47 °C) entsprechend einer Ausbeute von 96 % der Theorie. N-Ethylanilin wird nicht gefunden.

Beispiel 2

93 g (1,0 Mol) Anilin, 174 g (1,0 Mol) Dibutylcarbonat und 2 g Zirkontetrapropylat werden an der Kolonne zum Sieden erhitzt und Butanol abdestilliert. Nach 5-6 Stunden ist die Umsetzung vollständig und die Sumpftemperatur auf 190 °C gestiegen. Das Reaktionsprodukt kristallisiert beim Abkühlen. Nach Umkristallisieren aus Ligroin erhält man 172 g N-Phenyl-O-butylurethan, das sind 89 % der Theorie (Schmelzpunkt 60-61 °C).

Beispiel 3

93 g (1 Mol) Anilin, 356 g (3 Mol) Diethylcarbonat und 5 g Bleiacetat werden zum Sieden gebracht, und bei 135-136 °C im Sumpf wird über eine Kolonne Ethanol abgetrennt. Dabei langsam ausfallenden Diphenylharnstoff läßt man im Reaktionsgemisch. Nach 6 h erhöht man die Temperatur auf 180 °C unter Verwendung eines Druckgefäßes und hält noch 5 h bei dieser Temperatur. Durch fraktionierte Destillation erhält man N-Phenyl-O-ethylurethan in einer Ausbeute von 96 % der Theorie N-Ethylanilin wird nicht gefunden.

Beispiel 4

93 g Anilin, 356 g Diethylcarbonat und 3 g Zirkontetrapropylat werden 25 h unter Ethanolabspaltung auf 130-134 °C erhitzt. Durch fraktionierte Destillation gewinnt man N-Phenyl-O-ethylurethan in einer Ausbeute von 85 % der Theorie. Ein geringer Anteil von 1,5 % des eingesetzten Anilins geht in N-Ethylanilin über.

Beispiel 5

93 g Anilin, 356 g Diethylcarbonat und 5 g Zinkstearat werden nach 9 h Erhitzen auf 132-135 °C unter Ethanolabspaltung noch 4 h im Druckgefäß auf 180 °C gehalten. Man erhält eine Ausbeute an N-Phenyl-O-ethylurethan von 97 % der Theorie. N-Ethylanilin wird allenfalls spurenweise gefunden.

Beispiel 6

Es wird verfahren wie in Beispiel 1, als Katalysator wird Bleioxid verwendet. Die Reaktionszeit beträgt 22 h. Man entfernt den Katalysator durch Behandeln mit 20 g eines sulfonierten vernetzten Polystyrols und arbeitet durch Destillation auf. Man erhält 48 g N-Phenylethylurethan vom Schmelzpunkt 48-49 °C. Ausbeute : 88 % der Theorie.

Beispiel 7

24 g (0,33 Mol) n-Butylamin, 118 g (1 Mol) Diethylcarbonat und 1,7 g Titantetrabutylat werden wie in

Beispiel 1 umgesetzt. Nach 72 h Reaktionszeit arbeitet man durch Destillation auf. Bei 93-95 °C/11 Torr erhält man 24 g N-n-Butylethylurethan.

$n_D^{20}$ = 1,429 2. Ausbeute : 50 % der Theorie, bezogen auf eingesetztes n-Butylamin.

Der als Nebenprodukt entstandene Dibutylharnstoff kann analog den vorstehenden Beispielen durch weiteres Erhitzen des Reaktionsgemisches in N-Butylethylurethan umgewandelt werden.

Die Ausbeute an N-Butylethylurethan, bezogen auf das eingesetzte Butylamin, wird dadurch nahezu quantitativ.

Beispiel 8

30,5 g (0,25 Mol) 2,4-Toluylendiamin, 118 g (1 Mol) Diethylcarbonat und 2 g Titantetrabutylat werden an einer 50 cm-Mikrokolonne 40 h zum Rückfluß erhitzt, während bei 78-79 °C 23,5 g Ethanol abdestillieren. Man filtriert 32 g Polyharnstoff des Toluylendiamins ab und engt das Filtrat bis 80 °C/10 Torr ein. Man erhält durch Umkristallisieren aus Toluol 17 g 2,4-Toluylen-bisethylurethan vom Schmelzpunkt 136-137 °C. Ausbeute : 26 % der Theorie, bezogen auf eingesetztes m-Toluylendiamin.

Der neben dem Bisurethan erhaltene Polyharnstoff wird durch weiteres Erhitzen im Reaktionsgemisch in m-Toluylen-bisethylurethan übergeführt, so daß sich schließlich eine Ausbeute an Bisurethan, bezogen auf eingesetztes Toluylendiamin, von 94-97 % der Theorie ergibt.

Beispiel 9

19,8 g (0,1 Mol) 4,4'-Diaminodiphenylmethan, 69,6 g (0,4 Mol) Dibutylcarbonat und 2 g Titantetrabutylat werden an einer kleinen Kolonne zum Sieden erhitzt und n-Butanol abgespalten. Dann erhitzt man noch 12 h auf 190-195 °C, trennt vom ausgefallenen Polyharnstoff durch Aufnehmen des Reaktionsproduktes in Methylenchlorid und Filtration ab und destilliert Lösungsmittel und überschussiges Dibutylcarbonat im Vakuum ab. Man erhält als Rückstand 4,4'-Methylendiphenyl-bisbutylurethan (Schmelzpunkt 112-113 °C aus Toluol/Ligroin) in einer Ausbeute von 6,8 g entsprechend 17 % der Theorie.

Bei weiterer Umwandlung des Polyharnstoffes analog Beispiel 9 durch Erhitzen mit Dibutylcarbonat in Gegenwart von Titantetrabutylat erhöht sich die Ausbeute an 4,4'-Methylendiphenyl-bis-butylurethan auf 90-95 % der Theorie.

**Ansprüche**

1. Verfahren zur Herstellung von N,O-disubstituierten Urethanen durch Umsetzung von primären Aminen mit Dialkylcarbonaten in Gegenwart von Katalysatoren, dadurch gekennzeichnet, daß man als Katalysatoren neutrale oder basische anorganische oder organische Verbindungen des Bleis, Titans, Zinks oder Zirkons verwendet.

2. Verfahren gemäß Anspruch 1, dadurch gekennzeichnet, daß man als Amine primäre Mono- oder Polyamine mit aromatisch gebundenen primären Aminogruppen oder Gemische derartiger Verbindungen verwendet.

3. Verfahren gemäß Anspruch 1 und 2, dadurch gekennzeichnet, daß man als Amine Verbindungen oder Gemische von Verbindungen der Formel

$$R^1—(NH_2)_n$$

verwendet, in welcher

$R^1$ für einen gegebenenfalls Alkyl- und/oder Halogen-Substituenten und/oder Alkylen-Brücken aufweisenden aromatischen Kohlenwasserstoffrest mit insgesamt 6-15 Kohlenstoffatomen steht und

n für 1 oder 2 steht.

4. Verfahren gemäß Anspruch 1 bis 3, dadurch gekennzeichnet, daß man als Dialkylcarbonate Verbindungen der Formel

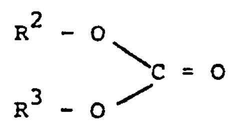

verwendet, in welcher

$R^2$ und $R^3$ für gleiche oder verschiedene Reste stehen und gesättigte aliphatische Kohlenwasserstoffreste mit 1 bis 4 Kohlenstoffatomen bedeuten.

**Claims**

1. A process for the production of N,O-disubstituted urethanes by reacting primary amines with

dialkyl carbonates in the presence of catalysts, characterised in that neutral or basis inorganic or organic compounds of lead, titanium, zinc or zirconium are used as the catalysts.

2. A process according to Claim 1, characterised in that primary monoamines or polyamines containing aromatically-bound, primary amino groups or mixtures of these compounds are used as the amines.

3. A process according to Claim 1 and 2, characterised in that the amines used are compounds or mixtures of compounds of the formula

$$R^1\text{—}(NH_2)_n$$

in which

$R^1$ represents an aromatic hydrocarbon radical with a total of 6-15 carbon atoms optionally containing alkyl and/or halogen substituents and/or alkylene bridges, and

n represents 1 or 2.

4. A process according to Claims 1 to 3, characterised in that compounds of the formula

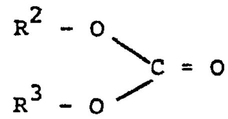

in which

$R^2$ and $R^3$ represent identical or different radicals and denote saturated aliphatic hydrocarbon radicals containing 1 to 4 carbon atoms are used as the dialkyl carbonates.

**Revendications**

1. Procédé pour la fabrication d'uréthannes N,O-disubstitués par réaction d'amines primaires avec des carbonates de dialkyles en présence de catalyseurs, caractérisé en ce que l'on utilise comme catalyseurs des composés inorganiques ou organiques, neutres ou basiques, du plomb, du titane, du zinc ou du zirconium.

2. Procédé selon la revendication 1, caractérisé en ce que l'on utilise comme amines primaires des mono- ou polyamines à groupes amino primaires à liaison aromatique ou des mélanges de ces composés.

3. Procédé selon les revendications 1 et 2, caractérisé en ce que l'on utilise comme amines des composés ou des mélanges de composés de formule

$$R^1\text{—}(NH_2)_n$$

dans laquelle

$R^1$ représente un reste d'hydrocarbure aromatique présentant éventuellement des substituants alkyles et/ou halogènes et/ou des ponts alkylènes, ayant un total de 6 à 15 atomes de carbone, et

n est égal à 1 ou 2.

4. Procédé selon les revendications 1 à 3, caractérisé en ce que l'on utilise comme carbonates de dialkyles des composés de formule

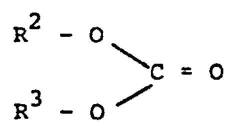

dans laquelle

$R^2$ et $R^3$ sont des restes identiques ou différents et représentent des restes d'hydrocarbures aliphatiques saturés en $C_1$-$C_4$.